(19) Europäisches Patentamt
European Patent Office
Office européen des brevets

(11) Numéro de publication: **0 363 252 A1**

# (12) DEMANDE DE BREVET EUROPEEN

(21) Numéro de dépôt: **89402629.3**

(22) Date de dépôt: **26.09.89**

(51) Int. Cl.5: **A61K 7/06 , C08G 77/38 , D06M 15/643**

Revendications pour les Etats contractants suivants: ES + GR.

(30) Priorité: **05.10.88 LU 87360**

(43) Date de publication de la demande: **11.04.90 Bulletin 90/15**

(84) Etats contractants désignés: **AT BE CH DE ES FR GB GR IT LI NL SE**

(71) Demandeur: **L'OREAL**
**14, Rue Royale**
**F-75008 Paris(FR)**

(72) Inventeur: **Chizat, François**
**27, rue Alexis Carrel**
**F-69500 Bron(FR)**
Inventeur: **Peignier, Michel**
**Rue du Joly Lentilly**
**F-69210 L'Arbresle(FR)**
Inventeur: **Grollier, Jean-François**
**16bis Boulevard Morland**
**F-75004 Paris(FR)**
Inventeur: **Dubief, Claude**
**9, rue Edmond Rostand**
**F-78150 Le Chesnay(FR)**

(74) Mandataire: **Casalonga, Axel et al**
**BUREAU D.A. CASALONGA - JOSSE**
**Morassistrasse 8**
**D-8000 München 5(DE)**

## (54) Emulsions d'organopolysiloxanes à fonction diester, leur application dans le traitement textile, cosmétique et dermatologique.

(57) L'invention concerne une émulsion silicone/eau comprenant, en milieu aqueux :

1°/ un organopolysiloxane à fonction diester présentant, par molécule, au moins un motif de formule :

$$ZR_aSiO_{\frac{3-a}{2}}$$

où Z a la formule :

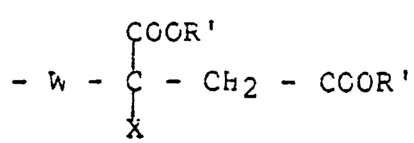

où les symboles R′ sont des radicaux hydrocarbonés saturés monovalents en $C_1$-$C_{12}$, alcoxyalkyle en $C_2$-$C_{12}$ ou aryle, alkylaryle ou arylalkyle en $C_6$-$C_{12}$, le symbole X est H ou $CH_3$, le symbole W est une liaison covalente ou un radical alkylène en $C_1$-$C_4$, les symboles R sont des radicaux alkyle en $C_1$-$C_{20}$, vinyle, phényle, trifluoro-3,3,3 propyle, hydroxyle, un seul des radicaux R par atome de silicium représentant hydroxyle, a est égal à 0, 1 ou 2;

2°/ un émulsionnant choisi parmi les tensio-actifs anioniques, cationiques, amphotères ou non-ioniques.

Application : au traitement des fibres textiles, à l'enduction des supports souples, au traitement cosmétique ou dermatologique des cheveux ou de la peau.

EP 0 363 252 A1

# Emulsions d'organopolysiloxanes à fonction diester, leur application dans le traitement textile, cosmétique et dermatologique.

La présente invention concerne des émulsions aqueuses d'organopolysiloxanes à fonction diester. Elle est le fruit d'une coopération entre les Sociétés L'OREAL et RHONE-POULENC CHIMIE.

Les émulsions, selon l'invention, sont utiles pour le traitement des fibres textiles synthétiques ou naturelles, des fibres kératiniques, telles que la laine ou les cheveux et pour l'enduction des supports souples, en particulier pour l'enduction de supports cellulosiques (papier). Ces émulsions se sont également révélées être particulièrement intéressantes dans le traitement cosmétique et dermatologique de la peau.

Les émulsions aqueuses conformes à l'invention comprennent, dans un milieu aqueux :

(i) au moins un organopolysiloxane à fonction diester présentant par molécule au moins un motif de formule :

$$ZR_aSiO_{\frac{3-a}{2}} \qquad (I)$$

dans laquelle :
Z est un radical de formule :

$$-W-\underset{\substack{| \\ X}}{\overset{\substack{COOR' \\ |}}{C}}-CH_2-COOR'$$

dans laquelle :
- les symboles R', identiques ou différents, sont choisis parmi les radicaux hydrocarbonés saturés, monovalents en $C_1$-$C_{12}$, les radicaux monovalents alcoxyalkyle en $C_2$-$C_{12}$ et les radicaux aryle, alkylaryle et arylalkyle en $C_6$-$C_{12}$;
- le symbole X est choisi parmi un atome d'hydrogène et le radical méthyle;
- le symbole W est choisi parmi une liaison covalente et un radical alkylène, linéaire ou ramifié, ayant de 1 à 4 atomes de carbone;
- les symboles R, identiques ou différents, sont choisis parmi les radicaux alkyle en $C_1$-$C_{20}$, vinyle, phényle et trifluoro-3,3,3 propyle, et un seul des radicaux R par atome de silicium peut être un hydroxyle;
- a est choisi parmi 0, 1 et 2;

(ii) une quantité efficace pour former une émulsion d'au moins un agent émulsionnant choisi parmi les tensio-actifs anioniques, non ioniques, cationiques, amphotères ou leurs mélanges.

Dans les motifs de formule (I) et en ce qui concerne le radical Z, W peut être choisi de préférence parmi les radicaux alkylène hydrocarbonés suivants :
- $CH_2$ - avec X = H
-$(CH_2)_3$ avec X = H

$$-CH_2-\underset{\substack{| \\ CH_3}}{CH}-CH_2- \text{ avec } X = H$$

Il peut également symboliser une simple liaison de valence et X désigne un méthyle.

Les radicaux R' peuvent être choisis parmi :
- les radicaux alkyle en $C_1$-$C_{12}$, tels que les radicaux méthyle, éthyle, n-propyle, isopropyle, n-butyle, éthyl-2 hexyle, heptyle et dodécyle;
- les radicaux aryle, alkylaryle et arylalkyle en $C_6$-$C_{12}$, tels que les radicaux phényle, benzyle et tolyle;
- les radicaux alcoxyalkyle en $C_2$-$C_{12}$, tels que méthoxyméthyle et éthoxyméthyle et méthoxy-2 éthyle.

Les radicaux R alkyle préférés sont choisis parmi les groupements méthyle, éthyle, propyle, n-butyle, n-octyle et éthyl-2 hexyle. De préférence, au moins 80% en nombre des radicaux R sont méthyle. Un seul

des radicaux R par atome de silicium peut être un hydroxyle, en particulier dans le cas où a vaut 2.

Les autres motifs siloxyle de l'organopolysiloxane répondent de préférence à la formule :

$R_bSiO_{\frac{4-b}{2}}$

dans laquelle :

R a la même signification que ci-dessus et b est égal à 0, 1, 2 ou 3.

De préférence, les organopolysiloxanes à fonction diester utilisés, selon l'invention, sont des polymères linéaires ou cycliques choisis parmi ceux de formule :

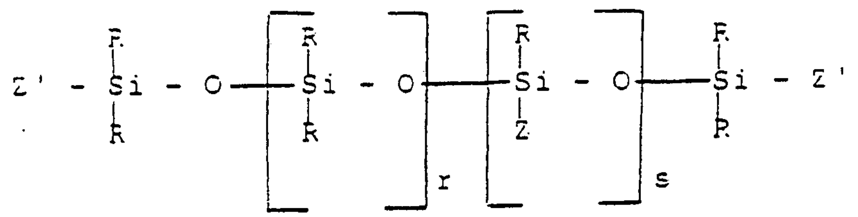

(II)

dans laquelle :

- les symboles R et Z ont la signification donnée ci-dessus;
- les symboles Z′, identiques ou différents, sont choisis parmi les radicaux R et Z;
- r est un nombre entier compris entre 0 et 500, inclusivement;
- s est un nombre entier choisi entre 0 et 50, inclusivement, et, si s vaut 0, au moins un des deux symboles Z′ est Z;

et ceux de formule :

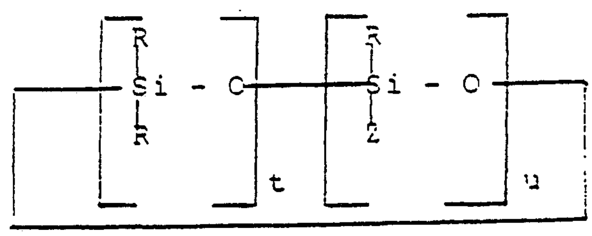

(III)

dans laquelle :

R et Z ont la même signification que ci-dessus;

u est un nombre entier compris entre 1 et 20 inclus; et

t est un nombre entier compris entre 0 et 20 inclus;

t+u est supérieur ou égal à 3.

On préfère plus particulièrement les polymères statistiques ou à blocs à motifs de formules (I), (II) et (III), présentant au moins l'une des caractéristiques suivantes :

- R et R′ sont méthyle;
- r est compris entre 5 et 50 inclus;
- s est compris entre 1 et 20 inclus;
- t+u est compris entre 3 et 10 inclus.

Les organopolysiloxanes à fonction diester peuvent être préparés, notamment, selon un procédé (A).

Au cours d'une première étape ($A_1$), on additionne un diester organique de formule :

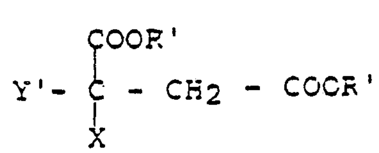

(IV)

ou de formule :

$$\begin{array}{l} CH_2 = C - COOR' \\ \quad\;\;\; | \\ \quad\;\;\; CH_2 - COOR' \end{array} \qquad (IVbis)$$

dans lesquelles :

Y′ est choisi parmi un groupe alcényle, linéaire ou ramifié, ayant de 2 à 4 atomes de carbone inclus;

R′ et X ont la même signification qu'à la formule (I),

sur un hydrogénoorganosilane de formule :

$HR_aSiCl_{3-a}$ (V)

dans laquelle R et a ont la même signification qu'à la formule (I) ci-dessus.

On obtient ainsi un produit d'addition de formule :

$ZR_aSiCl_{3-a}$ (VI)

dans laquelle R, Z et a ont la même signification qu'à la formule (I) ci-dessus.

L'étape ($A_1$) peut être effectuée en masse ou en solution dans un solvent organique.

La réaction est exothermique. On opère généralement sous reflux du mélange réactionnel à une température comprise entre 60 et 140 °C pendant une durée comprise entre 10 minutes et 3 heures.

On peut, soit couler le silane de formule (V) sur le diester de formule (IV) et/ou (IVbis), ou vice-versa, ou en même temps.

Il est préférable d'utiliser un excès molaire (de 10 à 50%) de silane de formule (V).

Il est préférable en vue d'augmenter la cinétique de réaction, d'opérer en présence d'un catalyseur. Les catalyseurs utilisables sont ceux utilisés pour faire la réaction d'hydrosilylation. Sont donc en particulier utilisables les peroxydes organiques, les radiations UV et les catalyseurs à base d'un métal du groupe du platine, en particulier le platine, le ruthénium et le rhodium à la dose de 10 à 500 ppm (calculés en poids de métal) par rapport au poids de silane de formule (V).

Comme exemples de catalyseurs, on peut citer le platine métal sur noir de carbone, les complexes platine/oléfine décrits dans les brevets américains US-A-3.159.601 et US-A-3.159.662, l'acide chloroplatinique, l'acide chloroplatineux, les complexes du platine et d'un vinylpolysiloxane, décrits dans le brevet américain US-A-3.419.593, les complexes du platine de degré voisin de zéro, décrits dans les brevets américains US-A-3.715.334, US-A-3.814.730 et les complexes de platine avec un produit organique à insaturation éthylénique, décrits dans les brevets européens EP-A-188.978 et EP-A-190.530.

En fin de réaction, les produits volatils sont éliminés par distillation sous vide. Le vide de la pompe à eau de 0,1 à 3 KPa est généralement suffisant.

Au cours d'une deuxième étape ($A_2$), on effectue l'hydrolyse (ou la cohydrolyse) et la polycondensation d'un silane de formule (VI).

Cette hydrolyse ou cohydrolyse et polycondensation peuvent être effectuées de préférence en phase aqueuse liquide en milieu acide (de préférence HCl) ou en milieu basique (de préférence $NH_4OH$) ou en milieu solvant dans des conditions similaires à celles de l'hydrolyse des chlorosilanes, telles que décrites aux pages 193 à 200 de l'ouvrage de NOLL "CHEMISTRY and TECHNOLOGY of SILICONES" Academic Press (1968).

La concentration en acide ou en base dans l'eau est comprise généralement entre 10 et 30% en poids. Le milieu d'hydrolyse comporte toujours au moins 2 moles d'eau par mole de silane, généralement de 10 à 100 moles d'eau. L'hydrolyse peut être effectuée en continu ou en discontinu à température ambiante (20 °C) ou à une température comprise entre 5 et 90 °C. L'hydrolyse peut être effectuée à pression égale ou supérieure à la pression atmosphérique en continu ou en discontinu avec, au moins pour le procédé continu, réinjection d'eau pour maintenir une phase aqueuse constante.

En vue d'obtenir les polymères de formules (II) et (III) ou leurs mélanges, on hydrolyse et polycondense les silanes de formule (VI) dans laquelle a = 1, en présence éventuellement d'un dichlorodiorganosilane de formule :

$R_2SiCl_2$ (VII)

dans laquelle R a la définition donnée à la formule (I) ci-dessus.

La polycondensation peut être arrêtée par simple neutralisation du milieu réactionnel. Dans ce cas, les polymères de formule (II) obtenus sont bloqués à chacune de leurs extrémités par un groupement hydroxyle (silanol) ou par le motif $R_2ZSiO_{0,5}$ si on utilise le silane $R_2ZSiCl$.

On peut également arrêter la polycondensation en ajoutant un composé organosilicié susceptible de réagir avec les hydroxyle terminaux, tels que les produits de formule :

$R_3$ Si-Cl; $R_3$ Si-NH-Si $R_3$ et $R_3$ Si-O-Si $R_3$

dans lesquelles les radicaux R ont la signification donnée à la formule (I) ci-dessus.

La durée de l'hydrolyse peut être comprise entre quelques secondes et plusieurs heures.

Après hydrolyse, la phase aqueuse est séparée de la phase siloxane par tout moyen physique approprié, généralement par décantation et extraction par un solvant organique comme l'éther isopropylique.

La phase siloxane peut être ultérieurement lavée à l'eau, puis distillée éventuellement pour séparer les polymères linéaires de formule (II) des polymères cycliques de formule (III).

Pour préparer les polymères de formules (I), (II) et (III), on peut également selon un deuxième procédé (B) à partir du polymère correspondant, dans lequel tous les radicaux Z et éventuellement Z′ sont des atomes d'hydrogène et par une réaction d'hydrosilylation, additionner un diester de formules (IV) ou (IVbis) définies ci-dessus.

Ce polymère est dénommé par la suite polymère à SiH; les groupes SiH peuvent être présents dans la chaîne et/ou aux extrémités de chaîne. Ces polymères à SiH sont des produits bien connus dans l'industrie des silicones et sont généralement disponibles dans le commerce.

Ils sont par exemple décrits dans les brevets américains US-A-3.220.972, US-A-3.697.473 et US-A-4.340.709.

Ce polymère à SiH peut donc être représenté par la formule :

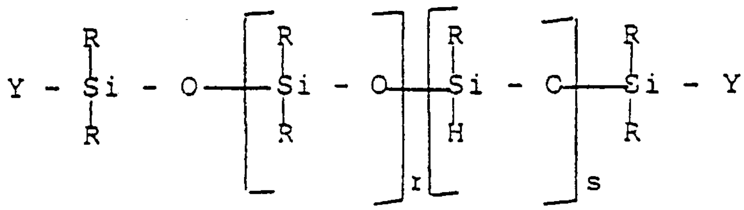

(VIII)

dans laquelle R, r et s ont la signification donnée ci-dessus pour la formule (II) et les radicaux Y, identiques ou différents, sont choisis parmi les radicaux R et un atome d'hydrogène;
et la formule :

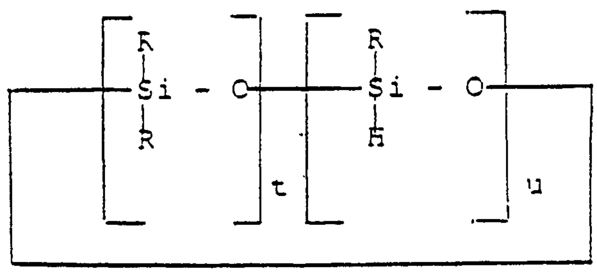

(IX)

dans laquelle R, t et u ont la signification donnée ci-dessus pour la formule (III).

Le procédé (E) met donc en oeuvre comme à l'étape ($A_1$) du procédé (A), une réaction d'hydrosilylation analogue et il est souhaitable d'effectuer cette réaction avec les mêmes catalyseurs que ceux indiqués à l'étape ($A_1$).

Cette réaction peut s'effectuer en masse ou au sein d'un solvant organique à une température comprise entre la température ordinaire (25° C) et 170° C.

Les produits volatils sont éliminés en fin de réaction par distillation sous vide et/ou par extraction.

Le procédé (A) permet d'obtenir des polymères de formule (II) présentant des groupes hydroxyle terminaux et des polymères de formules (II) et (III) présentant des radicaux R dont certains peuvent être des radicaux vinyle.

Le procédé (B) permet d'avoir des polymères de structure bien définie par le choix des polymères à SiH de départ.

Ces diorganopolysiloxanes à fonction diester se présentent généralement sous forme d'huiles plus ou moins visqueuses, de viscosité comprise entre 2 et 500.000 m.Pa.s, de préférence entre 5 et 5.000 m.Pa.s, à 25° C.

Comme produits de formule (IV), on préfère plus spécialement utiliser :

- les diesters de l'acide allylsuccinique ou methallylsuccinique éventuellement α-méthylé (formule (IV) dans laquelle Y′ est le groupement $CH_2=CH-CH_2-$ ou $CH_2=C(CH_3)-CH_2-$; X est H ou $CH_3$).

Les organopolysiloxanes modifiés obtenus par les procédés (A) et (B) ci-dessus sont également décrits dans les brevets américains US-A-4.207.246, US-A-4.322.473 et US-A-4.405.469 cités comme références.

- les diesters de l'acide itaconique (formule IVbis); on obtient alors deux formes isomères de radicaux Z :
$-CH_2CH(COOR')CH_2COOR'$ (formule I : W est $-CH_2-$, X est H); et
$-C(CH_3)(COOR')CH_2COOR'$ (formule I : W est une liaison de valence et X est méthyle).

Les émulsions selon la présente invention sont du type silicone dans l'eau (émulsion silicone/eau). Les agents tensio-actifs utilisables conformément à l'invention, sont par exemple décrits dans les brevets US-A-2.891.920, US-A-3.294.725, US-A-3.360.491, US-A-3.983.148 et FR-A-2.605.634 cités comme références.

Les émulsions silicone/eau, selon l'invention, comportent de préférence :

- 100 parties en poids d'un organopolysiloxane à fonction diester, choisi parmi ceux de formules (I), (II) et (III) ci-dessus;
- 1 à 300 parties en poids d'au moins un agent tensio-actif choisi parmi les agents tensio-actifs anioniques, cationiques, non-ioniques et amphotères;
- 5 à 2000 parties en poids d'eau.

Les agents tensio-actifs anioniques sont choisis parmi les alkylbenzènesulfonates de métaux alcalins, alkylsulfates de métaux alcalins, les alkyléthersulfates de métaux alcalins, les alkylaryléthersulfates de métaux alcalins et les dioctylsulfosuccinates de métaux alcalins.

Les agents tensio-actifs cationiques utilisés conformément à l'invention, sont choisis parmi les halogénures d'ammonium quaternaires, tels que de dialkyl($C_{10}$-$C_{30}$)diméthylammonium, d'alkyl($C_{10}$-$C_{30}$) triméthylammonium ou d'alkyl($C_{10}$-$C_{30}$)benzyldiméthyl ammonium et les sels d'ammonium quaternaires polyéthoxylés.

Les agents tensio-actifs amphotères utilisés conformément à l'invention sont choisis parmi les N-alkyl-($C_{10}$-$C_{22}$)bétaïnes, les N-alkyl($C_{10}$-$C_{22}$)sulfo bétaïnes, les N-alkyl($C_{10}$-$C_{22}$)amidobétaïnes, les alkyl($C_{10}$-$C_{22}$)imidazolines et les dérivés de l'asparagine.

Les agents tensio-actifs non ioniques sont choisis parmi les acides gras polyéthoxylés, les esters de sorbitan, les esters de sorbitan polyéthoxylés, les alkylphénols polyéthoxylés, les alcools gras polyéthoxylés, les amides gras polyéthoxylés ou polyglycérolés, les alcools et alphadiols polyglycérolés.

Pour préparer l'émulsion silicone/eau, divers procédés sont utilisables.

Selon un premier procédé, on mélange l'organopolysiloxane à fonction diester avec l'agent tensio-actif, ce dernier pouvant être déjà en solution aqueuse, puis on ajoute ensuite, si nécessaire, de l'eau. On transforme ensuite l'ensemble en une émulsion fine et homogène par passage dans un broyeur classique à colloïdes. Ce premier procédé est mis en oeuvre par exemple dans les brevets FR-A-2.064.563, FR-A-2.094.322, FR-A-2.114.230 et EP-A-169.098.

Selon un deuxième procédé, on prémélange l'agent tensio-actif et au moins une partie de l'eau à une température comprise entre la température ambiante (25 °C) et 80 °C jusqu'à l'obtention d'un mélange homogène, puis on ajoute lentement l'organopolysiloxane au prémélange, tout en agitant énergiquement à une température comprise entre 25 et 80 °C. On règle la consistance de l'émulsion homogène obtenue en ajoutant éventuellement de l'eau. Ce deuxième procédé est par exemple décrit dans les brevets français FR-A-2.471.210 et FR-A-2.485.923.

La demanderesse a constaté que les émulsions silicone/eau, conformes à la présente invention et préparées selon les deux procédés décrits précédemment, conféraient d'une manière surprenante une douceur améliorée aux fibres textiles naturelles et synthétiques, ainsi qu'aux fibres kératiniques.

Un objet de la présente invention est constitué par l'utilisation des émulsions silicone/eau telles que définies ci-dessus, pour le traitement des fibres textiles synthétiques ou naturelles et des fibres kératiniques et pour l'enduction des supports souples, en particulier pour l'enduction de supports cellulosiques, tels que le papier.

Ces émulsions sont par ailleurs utilisables en cosmétique et confèrent aux cheveux des propriétés particulièrement surprenantes de démêlage et de douceur et à la peau une douceur améliorée.

Les émulsions particulièrement préférées pour l'utilisation en cosmétique selon l'invention, renferment un diorganopolysiloxane à fonction diester défini ci-dessus, associé à un halogénure d'ammonium quaternaire.

Un autre objet de l'invention est donc constitué par une composition sous forme d'une émulsion aqueuse destinée au traitement des cheveux ou de la peau en cosmétique et/ou en dermatologie, contenant un diorganopolysiloxane à fonction diester et un halogénure d'ammonium quaternaire comportant au moins une chaîne grasse en $C_{10}$-$C_{30}$.

On utilise de préférence un halogénure de dialkyl($C_{10}$-$C_{30}$)diméthylammonium ou d'alkyl($C_{10}$-$C_{30}$) triméthylammonium.

On utilise plus particulièrement le chlorure de distéaryldiméthylammonium et le chlorure de béhényl

triméthylammonium.

Ces émulsions aqueuses à base de diorganopolysiloxane à fonction diester et d'halogénure d'ammonium quaternaire peuvent être préparées selon les procédés décrits ci-dessus ou selon un procédé consistant à diluer à l'eau ou dans de l'eau portée à une température de 70 à 100 °C, sous agitation, un cofondu de cette association.

Le cofondu est préparé par chauffage à une température légèrement supérieure au point de fusion du mélange des deux composants de l'association définis ci-dessus. Cette température est de préférence voisine de 90 °C.

Les proportions pondérales en chacun des composés en vue de réaliser le cofondu, peuvent être comprises entre 2,5 et 97,5% par rapport au poids total du confondu.

L'émulsion aqueuse est obtenue en diluant le cofondu progressivement sous agitation avec deux à dix fois et de préférence quatre à six fois son poids d'eau chaude, à une température voisine également du point de fusion des deux composants de l'association et de préférence à environ 90 °C.

L'émulsion aqueuse ainsi obtenue est particulièrement stable dans le temps et se dilue ensuite aisément dans l'eau froide.

Les compositions de traitement cosmétique renfermant l'émulsion aqueuse, stable conforme à l'invention et renfermant au moins un organopolysiloxane à fonction diester choisi parmi ceux de formules (I), (II) et (III) ci-dessus, sont plus particulièrement destinées à être appliquées sur les cheveux ou la peau. Dans leur forme de réalisation préférée, ils renferment l'halogénure d'ammonium quaternaire dans des proportions comprises entre 0,01 et 15% en poids et de préférence entre 0,02 et 10% en poids par rapport au poids total de la composition et le diorganopolysiloxane à fonction diester présentant par molécule un motif de formule (I) dans des proportions comprises entre 0,01 et 15% en poids et de préférence entre 0,02 et 10% en poids.

Ces compositions peuvent également renfermer un solvant et plus particulièrement un alcool en $C_1$-$C_4$ et se présenter sous forme épaissie ou non, de crème, de gel, et être pressurisées en aérosol sous forme de mousses et de sprays.

Elles peuvent plus particulièrement être utilisées comme shampooing, comme composition après-shampooing, comme produits à rincer applicables après-shampooing, avant ou après coloration et décoloration, avant ou après permanente ou défrisage, comme lotions de mise en plis ou de brushing, comme compositions restructurantes ou additifs aux permanentes ou comme compositions de soin pour la peau.

Elles peuvent également être utilisées en dermatologie, auquel cas, elles contiennent une substance active sur le plan dermatologique.

Les compositions conformes à l'invention peuvent contenir tous autres adjuvants habituellement utilisés en cosmétique, tels que des parfums, des colorants, des agents conservateurs, des agents hydratants, des agents séquestrants, des agents filtrants, des agents moussants, tels que les agents tensio-actifs et des polymères moussants, des agents de conditionnement, tels que par exemple des polymères et notamment, des polymères cationiques, anioniques, non-ioniques ou amphotères ou leurs mélanges, des agents épaississants, des stabilisateurs de mousse, des propulseurs ou autres adjuvants habituellement utilisés dans les compositions pour les cheveux ou la peau, suivant l'application envisagée.

Une forme de réalisation préférée consiste à associer dans la composition un polymère cationique et un polymère anionique. Le polymère anionique peut être choisi parmi les polymères dérivés d'acides carboxyliques comme par exemple l'acide acrylique ou méthacrylique, d'acide ou d'anhydride maléique, d'acide crotonique. Le polymère cationique comporte de préférence un grand nombre de groupements amine primaire, secondaire, tertiaire ou quaternaire et est plus particulièrement choisi parmi les polymères cationiques cellulosiques. Les polymères ont un poids moléculaire généralement compris entre 500 et 3.000.000.

De telles associations de polymères sont décrites entre autre dans le brevet français 2 383 660.

Leur pH est généralement compris entre 3 et 10. Il peut être ajusté à l'aide d'agents alcalinisants ou acidifiants bien connus dans la technique dans le domaine cosmétique.

Les épaississants peuvent être choisis parmi les gommes de xanthane, la gomme de guar ou ses dérivés, la gomme arabique ou de caroube, l'alginate de sodium, les dérivés de cellulose, les dérivés d'acide polyacrylique. Ces compositions peuvent également être épaissies par un mélange d'ester phosphorique et d'amide ou par un produit résultant de l'interaction ionique d'un polymère cationique constitué par un copolymère de cellulose ou d'un dérivé de cellulose greffé par un sel de monomère hydrosoluble d'ammonium quaternaire et d'un polymère anionique carboxylique ayant une viscosité capillaire absolue dans le diméthylformamide ou le méthanol, à une concentration de 5% et à 30 °C, inférieure ou égale à 30 x $10^{-3}$ Pa.s, tel que décrit plus particulièrement dans le brevet français n° 2 598 611.

Ces épaississants sont utilisés de préférence dans des proportions comprises entre 0,1 et 30% en

poids, et en particulier entre 0,2 et 15% en poids par rapport au poids total de la composition.

Les agents propulseurs sont des propulseurs classiques tels que plus particulièrement les alcanes, les fluoroalcanes, les chlorofluoroalcanes ou leurs mélanges.

Le procédé de traitement cosmétique mettant en oeuvre l'émulsion telle que définie ci-dessus, consiste essentiellement à appliquer la composition, soit sur les cheveux suivant l'usage envisagé (shampooing, traitement à rincer, traitement de coiffage sans rinçage), soit sur la peau (bain, douche, produits bronzants, produits pour le rasage, lotions parfumées, crèmes ou lait).

Les exemples suivants sont destinés à illustrer l'invention sans pour autant présenter un caractère limitatif.

Les exemples de référence sont destinés à illustrer la préparation des composés utilisés dans l'association conforme à l'invention.

## EXEMPLE DE REFERENCE 1

Dans un réacteur tricol de 2 litres équipé d'un réfrigérant, d'un agitateur et d'une ampoule de coulée, on charge 907 g, soit 5,74 moles d'itaconate de méthyle et 129 mg d'acide chloroplatinique ($H_2PtCl_6$).

On porte la température à 116° C puis on coule pendant 65 minutes 792,5 g (6,89 moles) de $CH_3HSiCl_2$, soit un excès molaire de 20% par rapport à l'itaconate.

La réaction étant exothermique, la température se maintient aux environs de 120° C sans addition supplémentaire de calories. En fin de coulée, la température est de 112° C. On maintient le mélange réactionnel au reflux durant 1 heure 50 minutes puis on distille l'excès de $CH_3HSiCl_2$ et on obtient 1274 g d'un adduct liquide dont le point d'ébullition est de 80° C sous 0,13 KPa. Le rendement pondéral en adduct est de 71%.

L'analyse RMN de l'adduct indique qu'il présente environ 60% molaire de radicaux :
$-CH_2CH(CCOCH_3)CH_2COOCH_3$
et 40% molaire de radicaux :
$-C(CH_3)(COOCH_3)CH_2COOCH_3$.

## EXEMPLE DE REFERENCE 2

Préparation d'un composé de formule (II) dans laquelle $R=CH_3$, les deux radicaux R terminaux = OH; $Z'=Z$ = mélange de radicaux $-CH_2-CH(COOCH_3)CH_2COOCH_3$ et $-C(CH_3)(COOCH_3)CH_2COOCH_3$; $r=0$; $s=6,6$ (valeur moyenne statistique).

Dans le même réacteur tricol que celui utilisé à l'exemple 1, on charge 340 g d'une solution aqueuse d'ammoniaque ($NH_4OH$ à 20% en poids) et 370 ml d'eau. On coule pendant 50 minutes 500 g (1,83 mole) de l'adduct obtenu à l'exemple 1 en solution dans 500 ml d'éther isopropylique, la température étant maintenue à 25° C.

En fin d'hydrolyse, on décante les eaux mères puis on ajoute de nouveau 500 ml d'éther isopropylique pour favoriser la décantation. On effectue de nouveau un lavage à l'eau de la solution organique décantée, on sèche et on dévolatilise jusqu'à une température de 100° C sous un vide de 2 KPa.

On obtient alors 323 g d'une huile limpide ayant une teneur pondérale en groupe hydroxyle de 1,8%, une viscosité à 25° C de 35 mPa.s et un pourcentage pondéral de fonction ester de 54,2%.

## EXEMPLE DE REFERENCE 3

Préparation d'un composé de formule (II) dans laquelle $R=CH_3$, $Z'=OH$; Z = mélange de radicaux $-CH_2CH(COOCH_3)CH_2COOCH_3$ et $-C(CH_3)(COOCH_3)CH_2COOCH_3$, r représente une valeur moyenne statistique égale à 40 et s représente une valeur moyenne statistique égale à 2.

Dans un réacteur tricol de 10 litres, on charge 2 800 g d'eau sur laquelle on coule pendant une heure 903 g (7 moles) de $(CH_3)_2SiCl_2$ et 98 g (0,35 mole) de l'adduct obtenu à l'exemple 1. Durant la coulée, la température s'élève graduellement de 25 à 65° C. Après la coulée, on maintient le mélange réactionnel sous agitation pendant 30 minutes et on décante les eaux acides. On ajoute 350 ml d'éther isopropylique, on effectue trois lavages et on concentre la solution éthérée suivant un premier palier jusqu'à 100° C à pression atmosphérique puis jusqu'à 80° C sous un vide de 2,5 KPa.

On obtient alors 456 g d'huile limpide et incolore ayant les caractéristiques suivantes :

| | |
|---|---|
| - viscosité à 25° C | 20 mPa.s |
| - % (pondéral) d'hydroxyle | 1 % |
| - pourcentage pondéral de fonction ester | 4,8 % |
| - rendement pondéral en huile | 77 % |

## EXEMPLE DE REFERENCE 4

Préparation d'un composé de formule (II) dans laquelle $Z' = R = CH_3$, Z = mélange de radicaux $-CH_2-CH(COOCH_3)CH_2COOCH_3$ et $C(CH_3)(COOCH_3)CH_2COOCH_3$; r = 31 (valeur exacte); s = 17 (valeur exacte).

On charge dans un réacteur tricol de 5 litres, 816 g d'une huile de formule :

$(CH_3)_3SiO(CH_3HSiO)_{17}[(CH_3)_2SiO]_{31}Si(CH_3)_3$

et 948 g d'itaconate de méthyle, le tout dans 1 540 g de xylène, ainsi que de l'acide chloroplatinique en quantité telle que l'on ait environ 150 ppm de platine métal par rapport au poids de polymère siloxane. On porte la température à 145° C que l'on maintient pendant 24 heures. Ensuite, on distille le xylène et l'itaconate de méthyle en excès par chauffage à 140° C sous un vide de 2,5 KPa.

On obtient alors 1 370 g d'une huile limpide et incolore de viscosité 950 mPa.s à 25° C et un pourcentage pondéral de fonction ester de 28,2%.

## EXEMPLE DE REFERENCE 5

Dans un réacteur de un litre, équipé d'un réfrigérant et d'une agitation, on charge en même temps :

- 174 g, soit 1,1 mole d'itaconate de méthyle,
- 46 g d'un hydrogénométhylsiloxane cyclique de pureté 92% de formule :

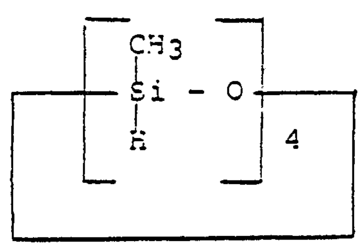

- 25 mg de $H_2PtCl_6(6H_2O)$

On porte le mélange réactionnel à 135° C et maintient la température entre 135 et 160° C pendant 1 heure 30 minutes.

On distille l'excès d'itaconate de méthyle par chauffage du mélange réactionnel à 160° C sous 0,133 KPa. On obtient 146 g d'huile dévolatilisée de couleur jaune orangée, de viscosité 2 240 mPa.s à 25° C, dont le pourcentage pondéral d'ester est de 50%.

Le spectre de masse et le spectre IR ($CHCl_3$) confirment que l'huile obtenue est bien le produit de formule :

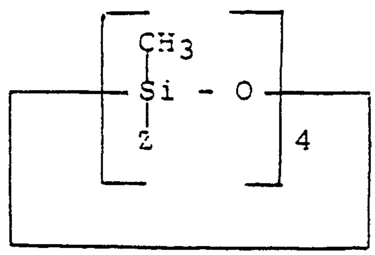

Z étant un radical itaconyle.

Les exemples 6 à 12 ci-après illustrent des émulsions utilisables dans le traitement des textiles. Dans ces exemples, on utilise l'huile silicone préparée à l'exemple de référence 3.

## EMULSIONS ANIONIQUES

### EXEMPLE 6

Tensio-actif : laurylsulfate de sodium.

Dans un bécher de 100 ml, on place 25 g d'huile de silicone et 2,5 g de laurylsulfate de Na. Sous agitation modérée à l'ultra Turrax®, on introduit lentement 4,5 g d'eau distillée puis on augmente la vitesse du Turrax® jusqu'à 10 000 t/mn. Lorsque la température atteint 65 à 70° C, la viscosité augmente beaucoup et on obtient une phase épaisse que l'on dilue progressivement à l'aide de 18 g d'eau distillée.

L'émulsion obtenue présente une granulométrie moyenne de 0,25 µm. Elle est stable dans le temps.

### EXEMPLE 7

Tensio-actif : dioctylsulfosuccinate

Dans un bécher de 100 ml, on place 2,17 g de dioctylsulfosuccinate (à 70% de matières sèches) et 15,12 g d'huile de silicone. L'ensemble forme, par agitation, une solution homogène. On voit que cette solution ne forme pas d'émulsion spontanée si on la verse goutte à goutte dans de l'eau distillée agitée magnétiquement.

Par agitation à l'ultra Turrax® et introduction progressive d'eau distillée, il y a augmentation progressive de la viscosité puis l'ensemble devient blanc pour 6,58 g d'eau ajoutée. On finit par une dilution à l'aide de 10,71 g d'eau distillée et obtient une émulsion stable dont la granulométrie moyenne est de 0,32 µm.

## EMULSIONS NON-IONIQUES

### EXEMPLE 8

Tensio-actif : nonylphénol éthoxylé.

Dans un bécher de 100 ml, on place 1,42 g de Cemulsol NP 5® (nonylphénol éthoxylé à 5 motifs oxyde d'éthylène), puis 1,63 g de Cemulsol NP 9® (nonylphénol éthoxylé à 9 motifs oxyde d'éthylène) et 5,62 g d'eau distillée.

Par agitation, on obtient une phase épaisse dans laquelle on entre progressivement, sous agitation à l'ultra Turrax®, 34,5 g d'huile de silicone. Ce broyat est alors dilué sous faible agitation par 28 g d'eau distillée.

L'émulsion ainsi obtenue présente une granulométrie moyenne de 1,25 µm. Cette émulsion présente après quelques jours une phase limpide au fond du flacon.

## EXEMPLE 9

Tensio-actif : dodécanediol polyglycérolé (à 3 motifs glycéryle).

Dans un bécher de 100 ml, on place 2,66 g de dodécanediol polyglycérolé (à 3 motifs glycéryle) et 24,4 g d'huile silicone. Sous agitation à l'ultra Turrax®, on introduit de l'eau distillée. Il ne se forme pas de phase épaisse, mais pour 11,2 g d'eau ajoutée, il y a inversion brutale. L'émulsion correspondante débulle difficilement mais est stable. La granulométrie moyenne est de 0,88 µm.

## EMULSIONS CATIONIQUES

## EXEMPLE 10

L'émulsion est réalisée comme à l'exemple 9 avec comme tensio-actifs :

. Cemulsol NP 9® (nonylphénol oxyéthylèné à 9OE) : 1,02 g
. Cemulsol NP 5® (nonylphénol oxyéthylèné à 5OE) : 0,48 g
. Cequartyl A® à 50% de matières sèches : 1,00 g (chlorure de dodécylbenzyldiméthylammonium)
. Huile silicone : 20,17 g

A l'ultra Turrax®, il y a inversion de phase pour 10,5 g d'eau distillée. La granulométrie moyenne est de 0,75 µm.

## TEST DE SUBSTANTIVITE

On prélève un morceau d'environ 3 g de tricot de coton teint d'environ 240 g/m$^2$. Après lavage avec un produit à base de dodécylbenzènesulfonate de sodium pour s'assurer de son caractère anionique et rinçage, on met cet article dans un bécher de 300 ml contenant 140 ml d'eau distillée acidifiée à pH 4 par de l'acide acétique et 0,1 g de l'émulsion précédente. Sous agitation magnétique, on chauffe pour monter la température de 1 °C par minute. Lorsque la température arrive à 42 °C, le bain est complètement épuisé (parfaitement limpide), ce qui a nécessité une vingtaine de minutes.

L'échantillon ainsi traité est séché à 70 °C. Son toucher est plus doux que le témoin.

## EXEMPLE 11

On procède comme à l'exemple 10, sauf que le tensio-actif utilisé est le Cequartyl A® à 50% de matière sèche utilisé à raison de 3,72 g de tensio-actif pour 15,20 g d'huile silicone.

Le test de substantivité montre que cette émulsion est plus substantive que la précédente, puisque l'épuisement du bain se fait dès 36 °C.

EXEMPLE 12

On procède comme à l'exemple 10. Le mélange à émulsionner a la composition suivante :
. Solution à 25% d'acétate de Cemulcat C® : 3,66 g (amine de coprah)
. Cemulsol NP 5® : 1,09 g
. Huile silicone : 15,20 g

L'inversion de phase se fait pour 6 g d'eau ajoutée.

L'émulsion est bulleuse même après ajout de 7 g d'eau.

Sa substantivité est bonne (40 ° C).

Les exemples 13 à 23 ci-après illustrent les compositions cosmétiques renfermant l'émulsion aqueuse à base d'organopolysiloxane à fonction diester et d'halogénure d'ammonium quaternaire.

EXEMPLE 13

On prépare une lotion capillaire à rincer à appliquer sur les cheveux après un shampooing, de composition suivante :

| | | |
|---|---|---|
| - Emulsion aqueuse à 20% MA obtenue par dilution à l'eau chaude d'un cofondu 50/50 en poids de chlorure de distéaryl-diméthylammonium et de diorganopoly-siloxane à fonction itaconate de diméthyle de l'exemple de référence 3 | | 2,0 g |
| (soit, en chlorure de distéaryl-diméthylammonium | | 0,2 g MA |
| et en diorganopolysiloxane | | 0,2 g MA) |
| - Eau | qsp | 100,0 g |

Sur des cheveux humides, notamment décolorés permanentés, cette lotion apporte une remarquable amélioration du démêlage par rapport à une lotion ne renfermant que du chlorure de distéaryldiméthylammonium.

Elle rend les cheveux séchés ou humides très doux sur toute leur longueur. Les cheveux séchés sont très brillants, gardent leur nervosité et ne présentent aucune électricité statique.

EXEMPLE 14

On prépare un après-shampooing capillaire de composition suivante :

- Emulsion aqueuse à 20% MA obtenue par dilution à l'eau chaude d'un cofondu 50/50 en poids de chlorure de béhényl triméthylammonium et de diorganopolysiloxane à fonction itaconate de diméthyle de l'exemple de référence 3 — 2,0 g
  (soit, en chlorure de béhényltriméthylammonium — 0,2 g MA
  et en diorganopolysiloxane — 0,2 g MA)
- Gomme de guar hydroxypropylée et quaternisée vendue sous la dénomination JAGUAR C 13 S par la Société CELANESE — 1,0 g
- Parfum, conservateur, colorant — qs
- Triéthanolamine — qs pH = 6,8
- Eau — qsp 100,0 g

On applique cette composition sur des cheveux lavés et essorés. Après rinçage, le démêlage des cheveux humides est très aisé. Les cheveux séchés sont nerveux, brillants et très doux.

## EXEMPLE 15

On prépare une lotion capillaire à rincer à appliquer sur les cheveux après un shampooing, de composition suivante :

| | |
|---|---|
| - Emulsion aqueuse à 20% MA obtenue par dilution à l'eau chaude d'un cofondu 50/50 en poids de chlorure de distéaryldiméthylammonium et de diorganopolysiloxane à fonction itaconate de diméthyle de l'exemple de référence 3 | 4,0 g |
| (soit, en chlorure de distéaryldiméthylammonium | 0,4 g MA |
| et en diorganopolysiloxane | 0,4 g MA) |
| - Condensat de l'épichlorhydrine sur le condensat d'acide adipique et de diéthylène triamine, préparé selon l'exemple (Ia) du brevet français 2 252 840 | 1,0 g MA |
| - Parfum, conservateur, colorant qs | |
| - Acide chlorhydrique qs pH = 8,5 | |
| - Eau qsp | 100,0 g |

Cette composition facilite le démêlage des cheveux humides et apporte aux cheveux séchés du gonflant et de la nervosité.

## EXEMPLE 16

On prépare un après-shampooing capillaire à rincer de composition suivante :

| | | |
|---|---|---|
| - Emulsion aqueuse à 20% MA obtenue par dilution à l'eau chaude d'un cofondu 50/50 en poids de chlorure de distéaryldiméthylammonium et de diorganopolysiloxane à fonction itaconate de diméthyle de l'exemple de référence 3 | | 10,0 g |
| (soit, en chlorure de distéaryldiméthyl-ammonium | | 1,0 g MA |
| et en diorganopolysiloxane | | 1,0 g MA) |
| - Hydroxyéthylcellulose greffée par du chlorure de diallyldiméthylammonium, vendue par la Société NATIONAL STARCH sous la dénomination CELQUAT L 200 | | 0,7 g MA |
| - Copolymère acide méthacrylique/ méthacrylate de méthyle 50/50 | | 0,7 g MA |
| - Parfum, conservateur, colorant qs | | |
| - Acide chlorhydrique qs pH = 7 | | |
| - Eau | qsp | 100,0 g |

Cette composition apporte un démêlage aisé sur cheveux mouillés et confère aux cheveux séchés du gonflant, de la nervosité, de la douceur et du brillant.

## EXEMPLE 17

On prépare un shampooing de composition suivante :

- Emulsion aqueuse à 20% MA obtenue par dilution à l'eau chaude d'un cofondu 50/50 en poids de chlorure de béhényl triméthylammonium et de diorganopolysiloxane à fonction itaconate de diméthyle de l'exemple de référence 3 — 4,0 g
  (soit, en chlorure de béhényl triméthylammonium — 0,4 g MA
  et en diorganopolysiloxane — 0,4 g MA)
- Tensio-actif amphotère dénommé "Cocoamphocarboxyglycinate" dans le Dictionnaire CTFA (3ème édition), vendu par la Société MIRANOL à la concentration de 38% de matière active (MA) sous la dénomination de MIRANOL $C_2M$ — 10,0 g
- Gomme de xanthane vendue sous la dénomination KELTROL T par la Société KELCO — 0,5 g
- Parfum, conservateur, colorant qs
- Acide chlorhydrique qs pH = 3,5
- Eau qsp 100,0 g

Ce shampooing aux bonnes propriétés moussantes permet de démêler les cheveux humides très facilement. Les cheveux, après séchage, sont brillants, souples et légers.

## EXEMPLE 18

On prépare un shampooing de composition suivante :

- Emulsion aqueuse à 20% MA obtenue par dilution à l'eau chaude d'un cofondu 50/50 en poids de chlorure de distéaryldiméthylammonium et de diorganopolysiloxane à fonction itaconate de diméthyle de l'exemple de référence 3 — 4,0 g
  (soit, en chlorure de distéaryldiméthylammonium — 0,4 g MA
  et en diorganopolysiloxane — 0,4 g MA)
- Tensio-actif de formule :

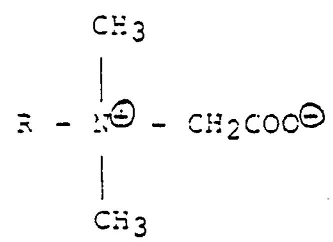

  R = radical coprah
  vendu à 30% de matière active (MA) par la Société HENKEL sous la dénomination DEHYTON AB 30 — 10,0 g
- Parfum, conservateur, colorant — qs
- Acide chlorhydrique — qs pH = 3
- Eau — qsp 100,0 g

Ce shampooing, aux bonnes propriétés moussantes, confère aux cheveux mouillés un excellent démêlage. Les cheveux, après séchage, sont doux et brillants.

## EXEMPLE 19

On prépare un gel fluide de coiffage à ne pas rincer, de composition suivante :

| | | |
|---|---|---|
| - Emulsion aqueuse à 20% MA obtenue par dilution à l'eau chaude d'un cofondu 50/50 en poids de chlorure de distéaryldiméthylammonium et de diorganopolysiloxane à fonction itaconate de diméthyle de l'exemple de référence 3 | | 0,2 g |
| (soit, en chlorure de distéaryldiméthyl-ammonium | | 0,02 g MA |
| et en diorganopolysiloxane | | 0,02 g MA) |
| - Copolymère acide méthacrylique/méthacrylate de méthyle 50/50 | | 0,7 g MA |
| - Hydroxyéthylcellulose greffée par du chlorure de diallyldiméthyl ammonium, vendue par la Société NATIONAL STARCH sous la dénomination CELQUAT L 200 | | 0,7 g MA. |
| - Alcool éthylique | qs | 10° |
| - Amino-2 méthyl-2 propanol-1 qs pH = 7,5 | | |
| - Parfum, colorant, conservateur qs | | |
| - Eau | qsp | 100,0 g |

Appliquée sur des cheveux humides, cette composition apporte un démêlage aisé et de la douceur aux cheveux. Les cheveux séchés sont brillants et la coiffure de bonne tenue.

## EXEMPLE 20

On prépare une mousse de coiffage de composition suivante :

| | |
|---|---|
| - Emulsion aqueuse à 20% MA obtenue par dilution à l'eau chaude d'un cofondu 50/50 en poids de chlorure de distéaryldiméthylammonium et de diorganopolysiloxane à fonction itaconate de diméthyle de l'exemple de référence 3 | 0,2 g |
| (soit, en chlorure de distéaryldiméthyl-ammonium | 0,02 g MA |
| et en diorganopolysiloxane | 0,02 g MA) |
| - Hydroxyéthylcellulose greffée par du chlorure de diallyldiméthylammonium, vendue par la Société NATIONAL STARCH sous la dénomination CELQUAT L 200 | 0,4 g |
| - Copolymère acide méthacrylique/méthacrylate d'hexyle 82/18 | 0,4 g MA |
| - Alcool éthylique | qs 10° |
| - Amino-2 méthyl-2 propanol-1 | qs pH = 7,5 |
| - Parfum, colorant, conservateur | qs |
| - Eau | qsp 100,0 g |

| Conditionnement aérosol : | |
|---|---|
| Composition ci-dessus<br>Fréons 12/114 (57/43) | 90 g<br>10 g<br>Total 100 g |

On applique cette mousse sur les cheveux lavés et essorés. On ne rince pas et l'on sèche. Les cheveux se démêlent facilement, sont doux et brillants.

## EXEMPLE 21

On prépare une crème traitante capillaire à rincer en cofondant vers 90 °C un mélange pondéral 50/50 des deux composés suivants :

| | |
|---|---|
| - Chlorure de distéaryldiméthylammonium | 50,0 g |
| - Diorganopolysiloxane à fonction itaconate de diméthyle de l'exemple de référence 4 | 50,0 g |

qu'on dilue ensuite peu à peu en agitant avec cinq fois son poids en eau chaude (à 90 °C).

On obtient une crème à 20% de MA dont on ajuste le pH à 3,5 avec HCl et addition du parfum

conservateur et colorant.

Appliquée sur les cheveux lavés et essorés, les cheveux étant ensuite rincés, cette crème apporte un démêlage remarquable, beaucoup de douceur, de brillance et de souplesse.

EXEMPLE 22

On prépare une lotion traitante capillaire non rincée de composition suivante :

| | | |
|---|---|---|
| - Emulsion aqueuse à 20% MA obtenue par dilution à l'eau chaude d'un cofondu 50/50 en poids de chlorure de distéaryldiméthylammonium et de diorganopolysiloxane à fonction itaconate de diméthyle de l'exemple de référence 2 | | 0,5 g |
| (soit exprimé en chlorure de distéaryl-diméthylammonium | | 0,05 g MA |
| et en diorganopolysiloxane | | 0,05 g MA) |
| - Parfum, colorant, conservateur | qs | |
| - Triéthanolamine | qs pH = 4,9 | |
| - Eau | qsp | 100,0 g |

On applique cette lotion sur des cheveux lavés et essorés.

Après séchage, ce traitement facilite le passage de la brosse au brushing et confère aux cheveux du brillant et de la douceur.

EXEMPLE 23

On prépare une crème protectrice pour la peau de composition suivante :

- Emulsion aqueuse à 20% MA obtenue par dilution à l'eau chaude d'un cofondu 30/70 en poids de chlorure de distéaryldiméthylammonium et de diorganopolysiloxane à fonction itaconate de diméthyle de l'exemple de référence 3 — 50,0 g
  (soit exprimé en chlorure de distéaryl-diméthylammonium — 3,0 g MA
  et en diorganopolysiloxane — 7,0 g MA)
- Colorant, conservateur, parfum qs
- Triéthanolamine qs pH = 4,5
- Eau qsp 100,0 g

Cette crème hydratante apporte douceur et souplesse à la peau.

## Revendications

1. Emulsion silicone/eau, caractérisée par le fait qu'elle comporte en milieu aqueux :

1°/un organopolysiloxane à fonction diester présentant, par molécule, au moins un motif de formule (I) suivante :

$$ZR_aSiO_{\frac{3-a}{2}} \quad (I)$$

dans laquelle :
Z est un radical de formule :

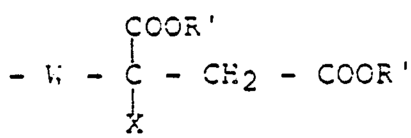

dans laquelle :
. les symboles R′, identiques ou différents, sont choisis parmi les radicaux hydrocarbonés saturés monovalents en $C_1$-$C_{12}$, les radicaux alcoxyalkyle en $C_2$-$C_{12}$ et les radicaux aryle, alkylaryle et arylalkyle en $C_6$-$C_{12}$;
. le symbole X est choisi parmi un atome d'hydrogène et le radical méthyle;
. le symbole W est choisi parmi une liaison covalente et un radical alkylène, linéaire ou ramifié, ayant de 1 à 4 atomes de carbone;
. les symboles R, identiques ou différents, sont choisis parmi les radicaux alkyle en $C_1$-$C_{20}$, vinyle, phényle et trifluoro-3,3,3 propyle, hydroxyle, à la condition qu'un seul des radicaux R par atome de silicium soit un hydroxyle;
a est choisi parmi 0, 1 et 2;

2°/une quantité efficace pour former une émulsion d'au moins un émulsionnant choisi parmi les agents tensio-actifs anioniques, cationiques, amphotères et non-ioniques.

2. Emulsion selon la revendication 1, caractérisée par le fait que les agents tensio-actifs anioniques sont choisis parmi les alkylbenzène sulfonates, les alkylsulfates, les alkyléthersulfates, les alkylaryléthersulfates et les dioctylsulfosuccinates de métaux alcalins.

3. Emulsion selon la revendication 1, caractérisée par le fait que les agents tensio-actifs cationiques sont choisis parmi les halogénures d'ammonium quaternaires et les sels d'ammonium quaternaires

polyéthoxylés.

4. Emulsion selon la revendication 1, caractérisée par le fait que les agents tensio-actifs amphotères sont choisis parmi les N-alkyl($C_{10}$-$C_{22}$) bétaïnes, les N-alkyl($C_{10}$-$C_{22}$)sulfobétaïnes, les N-alkyl($C_{10}$-$C_{22}$)-amidobétaïnes, les alkyl($C_{10}$-$C_{22}$) imidazolines et les dérivés de l'asparagine.

5. Emulsion selon la revendication 1, caractérisée par le fait que les agents tensio-actifs non-ioniques sont choisis parmi les acides gras polyéthoxylés, les esters de sorbitan, les esters de sorbitan polyéthoxylés, les alkylphénols polyéthoxylés, les alcools gras polyéthoxylés, les amides gras polyéthoxylés ou polyglycérolés, les alcools et α-diols polyglycérolés.

6. Emulsion selon l'une quelconque des revendications 1 à 5, caractérisée par le fait que dans la formule (I), les symboles W et X sont choisis parmi :

. W est $CH_2$ et X est H;

. W est $-(CH_2)_3-$ et X est H;

. W est 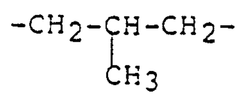 et X est H;

. W est une liaison de valence et X est méthyle.

7. Emulsion selon l'une quelconque des revendications 1 à 6, caractérisée par le fait que l'organopolysiloxane à fonction diester est choisi parmi ceux de formule :

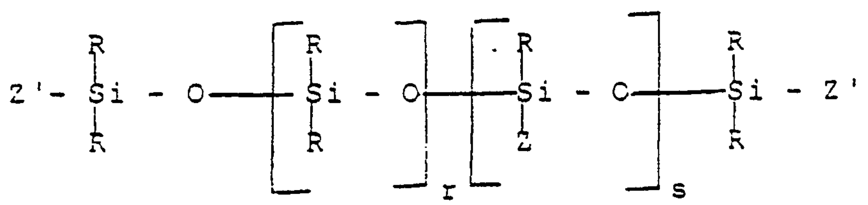 (II)

dans laquelle :

- les symboles R et Z ont la signification donnée dans la revendication 1;
- les symboles Z′, identiques ou différents, sont choisis parmi les radicaux R et Z;
- r est un nombre entier compris entre C et 500 inclusivement;
- s est un nombre entier choisi entre 0 et 50 inclusivement, et, si s est 0, au moins un des deux symboles Z′ est Z;

et ceux de formule :

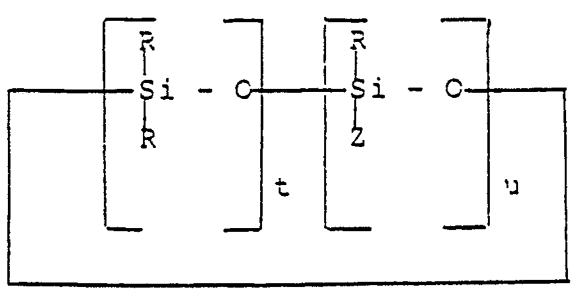 (III)

dans laquelle :

R et Z ont la même signification que dans la revendication 1;

u est un nombre entier compris entre 1 et 20 inclus; et

t est un nombre entier compris entre 0 et 20 inclus;

t+u est supérieur ou égal à 3.

8. Emulsion selon la revendication 7, caractérisée par le fait que dans la formule de l'organosiloxane à fonction diester :

- R et R′ sont méthyle;
- r est compris entre 5 et 50 inclus;
- s est compris entre 1 et 20 inclus;

- t+u est compris entre 3 et 10 inclus.

9. Emulsion selon l'une quelconque des revendications précédentes, caractérisée par le fait qu'elle comporte :

- 100 parties en poids de l'organopolysiloxane à fonction diester;
- 1 à 300 parties en poids d'au moins un agent tensio-actif;
- 5 à 2 000 parties d'eau.

10. Utilisation de l'émulsion telle que définie dans l'une quelconque des revendications 1 à 9, pour le traitement des fibres textiles naturelles ou synthétiques.

11. Utilisation de l'émulsion telle que définie dans l'une quelconque des revendications 1 à 9, pour le traitement des fibres kératiniques.

12. Utilisation de l'émulsion telle que définie dans l'une quelconque des revendications 1 à 9, pour l'enduction de supports souples, en particulier l'enduction de supports cellulosiques.

13. Utilisation de l'émulsion telle que définie dans l'une quelconque des revendications 1 à 9, pour le traitement cosmétique ou dermatologique des cheveux ou de la peau.

14. Composition cosmétique destinée au traitement des cheveux ou de la peau et se présentant sous forme d'une émulsion aqueuse silicone/eau, caractérisée par le fait que l'émulsion est telle que définie dans l'une quelconque des revendications 1 à 9.

15. Composition selon la revendication 14, caractérisée par le fait qu'elle comprend :

(i) au moins un organopolysiloxane à fonction diester présentant par molécule au moins un motif de formule (I) suivante :

$$ZR_aSiO_{\frac{3-a}{2}} \quad (I)$$

dans laquelle :

Z est un radical de formule :

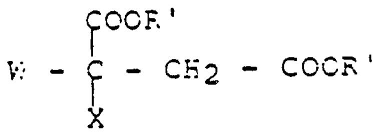

dans laquelle :

. les symboles R′, identiques ou différents, sont choisis parmi les radicaux hydrocarbonés saturés monovalents en $C_1$-$C_{12}$, les radicaux alcoxyalkyle en $C_2$-$C_{12}$, et les radicaux aryle, alkylaryle et arylalkyle en $C_6$-$C_{12}$;

. le symbole X est choisi parmi un atome d'hydrogène et le radical méthyle;

. le symbole W est choisi parmi une liaison covalente et un radical alkylène, linéaire ou ramifié, ayant de 1 à 4 atomes de carbone;

. les symboles R, identiques ou différents, sont choisis parmi les radicaux alkyle en $C_1$-$C_{20}$, vinyle, phényle et trifluoro-3,3,3 propyle, hydroxyle, à la condition qu'un seul des radicaux R par atome de silicium soit un hydroxyle;

(ii) et au moins un halogénure d'ammonium quaternaire comportant au moins une chaîne grasse en $C_{10}$-$C_{30}$.

16. Composition selon la revendication 15, caractérisée par le fait que l'halogénure d'ammonium quaternaire est choisi parmi le chlorure de distéaryldiméthylammonium et le chlorure de béhényl triméthylammonium.

17. Composition selon la revendication 15, caractérisée par le fait qu'elle renferme dans un milieu aqueux 0,01 à 15% en poids d'halogénure d'ammonium quaternaire dont un au moins des groupements alkyle est un groupement en $C_{10}$-$C_{30}$ et au moins un polyorganosiloxane tel que défini dans l'une quelconque des revendications 1, 6, 7 et 8, dans des proportions comprises entre 0,01 et 15% et de préférence entre 0,02 et 10% en poids.

18. Composition selon l'une quelconque des revendications 14 à 17, caractérisée par le fait qu'elle contient également un solvant cosmétiquement acceptable choisi parmi les alcools en $C_1$-$C_4$.

19. Composition selon l'une quelconque des revendications 14 à 18, caractérisée par le fait qu'elle contient en plus au moins un adjuvant cosmétiquement acceptable choisi parmi les parfums, les colorants, les agents conservateurs, les produits hydratants, les agents séquestrants, les agents filtrants solaires, les agents moussants, les agents de conditionnement, les agents épaississants, les stabilisateurs de mousse, les propulseurs.

20. Composition selon l'une quelconque des revendications 14 à 19, caractérisée par le fait qu'elle contient en plus, au moins un polymère cationique et au moins un polymère anionique ayant un poids moléculaire compris entre 500 et 3.000.000.

21. Composition selon l'une quelconque des revendications 14 à 20, caractérisée par le fait que son pH est ajusté entre 3 et 10.

22. Composition selon l'une quelconque des revendications 14 à 21, caractérisée par le fait qu'elle contient des épaississants choisis parmi la gomme de xanthane, la gomme de guar ou ses dérivés, la gomme arabique ou la gomme de caroube, l'alginate de sodium, les dérivés de cellulose, les dérivés d'acide polyacrylique, des mélanges d'acide phosphorique et d'amides; ou bien le produit résultant de l'interaction ionique d'un polymère cationique constitué par un copolymère de cellulose ou d'un dérivé de cellulose greffé par un sel de monomère hydrosoluble d'ammonium quaternaire et d'un polymère anionique carboxylique ayant une viscosité capillaire absolue dans le diméthylformamide ou le méthanol, à une concentration de 5% et à 30° C, égale ou inférieure à 30 x $10^{-3}$ Pa.s.

23. Application de la composition telle que définie dans l'une quelconque des revendications 14 à 22, comme shampooing, comme produit après-shampooing, produit à rincer appliqué avant ou après shampooing, avant ou après coloration ou décoloration, avant ou après permanente ou défrisage, comme lotion de mise en plis ou de brushing, comme composition restructurante, comme additif aux permanentes.

24. Procédé de traitement cosmétique des cheveux ou de la peau, caractérisé par le fait que l'on applique sur les cheveux ou sur la peau au moins une composition telle que définie dans l'une quelconque des revendications 14 à 22.

25. Composition dermatologique, caractérisée par le fait qu'elle se présente sous la forme d'une émulsion aqueuse silicone/eau telle que définie dans l'une quelconque des revendications 1 à 9 et qu'elle contient, en outre, une substance active dermatologique.

Revendications pour l'Etat contractant suivant: ES

1. Procédé de préparation d'une émulsion silicone/eau, caractérisé par le fait que l'on mélange :

1°/100 parties en poids d'un organopolysiloxane à fonction diester présentant, par molécule, au moins un motif de formule (I) suivante :

$ZR_aSiO_{\frac{3-a}{2}}$ (I)

dand laquelle :

Z est un radical de formule :

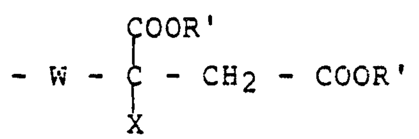

dans laquelle :

. les symboles R', identiques ou différents, sont choisis parmi les radicaux hydrocarbonés saturés monovalents en $C_1$-$C_{12}$, les radicaux alcoxyalkyle en $C_2$-$C_{12}$ et les radicaux aryle, alkylaryle et arylalkyle en $C_6$-$C_{12}$;

. le symbole X est choisi parmi un atome d'hydrogène et le radical méthyle;

. le symbole W est choisi parmi une liaison covalente et un radical alkylène, linéaire ou ramifié, ayant de 1 à 4 atomes de carbone;

. les symboles R, identiques ou différents, sont choisis parmi les radicaux alkyle en $C_1$-$C_{20}$, vinyle, phényle et trifluoro-3,3,3 propyle, hydroxyle, à la condition qu'un seul des radicaux R par atome de silicium soit un hydroxyle;

a est choisi parmi 0, 1 et 2;

2°/1 à 300 parties en poids d'au moins un agent tensio-actif anionique, cationique, amphotère ou non-ionique;

3°/5 à 2000 parties d'eau.

2. Procédé de préparation d'une émulsion selon la revendication 1, caractérisé par le fait que les agents tensio-actifs anioniques sont choisis parmi les alkylbenzènesulfonates, les alkylsulfates, les alkyléthersulfates, les alkylaryléthersulfates et les dioctylsulfosuccinates de métaux alcalins.

3. Procédé de préparation d'une émulsion selon la revendication 1, caractérisé par le fait que les agents

tensio-actifs cationiques sont choisis parmi les halogénures d'ammonium quaternaires et les sels d'ammonium quaternaires polyéthoxylés.

4. Procédé selon la revendication 1, caractérisé par le fait que les agents tensio-actifs amphotères sont choisis parmi les N-alkyl($C_{10}$-$C_{22}$) bétaïnes, les N-alkyl($C_{10}$-$C_{22}$)sulfobétaïnes, les N-alkyl($C_{10}$-$C_{22}$)-amidobétaïnes, les alkyl($C_{10}$-$C_{22}$) imidazolines et les dérivés de l'asparagine.

5. Procédé selon la revendication 1, caractérisé par le fait que les agents tensio-actifs non-ioniques sont choisis parmi les acides gras polyéthoxylés, les esters de sorbitan, les esters de sorbitan polyéthoxylés, les alkylphénols polyéthoxylés, les alcools gras polyéthoxylés, les amides gras polyéthoxylés ou polyglycérolés, les alcools et $\alpha$-diols polyglycérolés.

6. Procédé selon l'une quelconque des revendications 1 à 5, caractérisé par le fait que dans la formule (I), les symboles W et X sont choisis parmi :

. W est $CH_2$ et X est H;

. W est $-(CH_2)_3-$ et X est H;

. W est 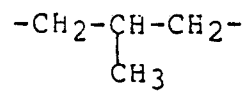 et X est H;

. W est une liaison de valence et X est méthyle.

7. Procédé selon l'une quelconque des revendications 1 à 6, caractérisé par le fait que l'organopolysiloxane à fonction diester est choisi parmi ceux de formule :

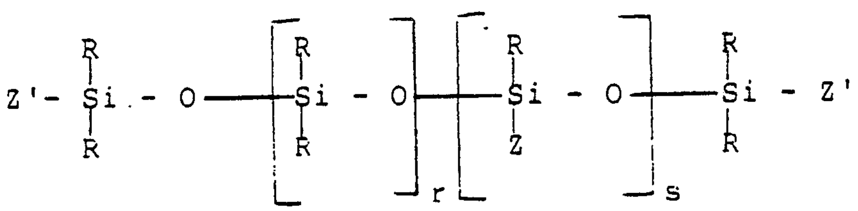

(II)

dans laquelle :

- les symboles R et Z ont la signification donnée dans la revendication 1;
- les symboles Z', identiques ou différents, sont choisis parmi les radicaux R et Z;
- r est un nombre entier compris entre 0 et 500 inclusivement;
- s est un nombre entier choisi entre 0 et 50 inclusivement, et, si s est 0, au moins un des deux symboles Z' est Z;

et ceux de formule :

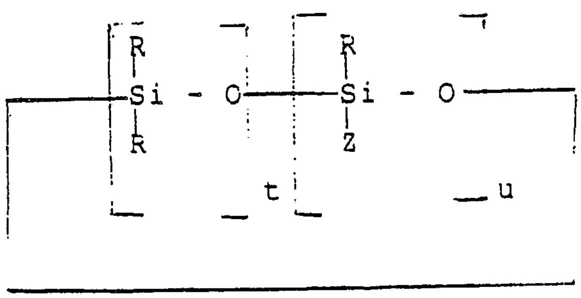

(III)

dans laquelle :

R et Z ont la même signification que dans la revendication 1;

u est un nombre entier compris entre 1 et 20 inclus; et

t est un nombre entier compris entre 0 et 20 inclus;

t + u est supérieur ou égal à 3.

8. Procédé selon la revendication 7, caractérisé par le fait que dans la formule de l'organosiloxane à fonction diester :

- R et R' sont méthyle;
- r est compris entre 5 et 50 inclus;

- s est compris entre 1 et 20 inclus;
- t + u est compris entre 3 et 10 inclus.

9. Procédé de préparation d'une composition cosmétique destinée au traitement des cheveux ou de la peau et se présentant sous forme d'une émulsion aqueuse silicone/eau, caractérisé par le fait que l'émulsion est telle que préparée dans le procédé selon l'une quelconque des revendication 1 à 8.

10. Composition destinée au traitement des cheveux ou de la peau sous forme d'émulsion aqueuse silicone/eau, caractérisée par le fait qu'elle comprend :

(i) au moins un organopolysiloxane à fonction diester présentant par molécule au moins un motif de formule (I) suivante :

$ZR_aSiO_{\frac{3-a}{2}}$ (I)

dans laquelle :

Z est un radical de formule :

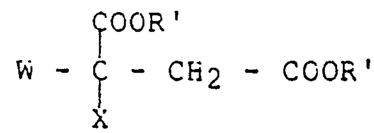

dans laquelle :

. les symboles R', identiques ou différents, sont choisis parmi les radicaux hydrocarbonés saturés monovalents en $C_1$-$C_{12}$, les radicaux alcoxyalkyle en $C_2$-$C_{12}$, et les radicaux aryle, alkylaryle et arylalkyle en $C_6$-$C_{12}$;

. le symbole X est choisi parmi un atome d'hydrogène et le radical méthyle;

. le symbole W est choisi parmi une liaison covalente et un radical alkylène, linéaire ou ramifié, ayant de 1 à 4 atomes de carbone;

. les symboles R, identiques ou différents, sont choisis parmi les radicaux alkyle en $C_1$-$C_{20}$, vinyle, phényle et trifluoro-3,3,3 propyle, hydroxyle, à la condition qu'un seul des radicaux R par atome de silicium soit un hydroxyle;

(ii) et au moins un agent tensio-actif anionique, cationique, amphotère ou non-ionique.

11. Composition selon la revendication 10, caractérisée par le fait que l'agent tensio-actif cationique est un halogénure d'ammonium quaternaire comportant au moins une chaîne grasse en $C_{10}$-$C_{30}$.

12. Composition selon la revendication 10, caractérisée par le fait que l'halogénure d'ammonium quaternaire est choisi parmi le chlorure de distéaryldiméthylammonium et le chlorure de béhényl triméthylammonium.

13. Composition selon la revendication 10, caractérisée par le fait qu'elle renferme dans un milieu aqueux 0,01 à 15% en poids d'halogénure d'ammonium quaternaire dont un au moins des groupements alkyle est un groupement en $C_{10}$-$C_{30}$ et au moins un polyorganosiloxane tel que défini dans l'une quelconque des revendications 1, 6, 7 et 8, dans des proportions comprises entre 0,01 et 15% et de préférence entre 0,02 et 10% en poids.

14. Composition selon l'une quelconque des revendications 10 à 13, caractérisée par le fait qu'elle contient également un solvant cosmétiquement acceptable choisi parmi les alcools en $C_1$-$C_4$.

15. Composition selon l'une quelconque des revendications 10 à 14, caractérisée par le fait qu'elle contient en plus au moins un adjuvant cosmétiquement acceptable choisi parmi les parfums, les colorants, les agents conservateurs, les produits hydratants, les agents séquestrants, les agents filtrants solaires, les agents moussants, les agents de conditionnement, les agents épaississants, les stabilisateurs de mousse, les propulseurs.

16. Composition selon l'une quelconque des revendications 10 à 15, caractérisée par le fait qu'elle contient en plus, au moins un polymère cationique et au moins un polymère anionique ayant un poids moléculaire compris entre 500 et 3.000.000.

17. Composition selon l'une quelconque des revendications 10 à 16 , caractérisée par le fait que son pH est ajusté entre 3 et 10.

18. Composition selon l'une quelconque des revendications 10 à 17, caractérisée par le fait qu'elle contient des épaississants choisis parmi la gomme de xanthane, la gomme de guar ou ses dérivés, la gomme arabique ou la gomme de caroube, l'alginate de sodium, les dérivés de cellulose, les dérivés d'acide polyacrylique, des mélanges d'acide phosphorique et d'amides; ou bien le produit résultant de l'interaction ionique d'un polymère cationique constitué par un copolymère de cellulose ou d'un dérivé de

cellulose greffé par un sel de monomère hydrosoluble d'ammonium quaternaire et d'un polymère anionique carboxylique ayant une viscosité capillaire absolue dans le diméthylformamide ou le méthanol, à une concentration de 5% et à 30° C, égale ou inférieure à 30 x $10^{-3}$ Pa.s.

19. Composition selon l'une quelconque des revendications 10 à 18, caractérisée par le fait qu'elle se présente sous la forme d'un shampooing, d'un produit après-shampooing, d'un produit à rincer appliqué avant ou après shampooing, avant ou après coloration ou décoloration, avant ou après permanente ou défrisage, d'une lotion de mise en plis ou de brushing, d'une composition restructurante ou d'un additif aux permanentes.

20. Composition destinée au traitement des fibres textiles naturelles ou synthétiques se présentant sous forme d'une émulsion silicone/eau, caractérisée par le fait que l'émulsion est telle que préparée dans le procédé selon l'une quelconque des revendications 1 à 8.

21. Composition destinée à l'enduction des supports souples et en particulier des supports cellulosiques se présentant sous forme d'émulsion silicone/eau, caractérisée par le fait que l'émulsion est telle que préparée dans le procédé selon l'une quelconque des revendications 1 à 8.

22. Procédé de préparation d'une composition dermatologique se présentant sous forme d'émulsion silicone/eau, caractérisé par le fait que l'on introduit dans une émulsion telle que préparée dans le procédé selon l'une quelconque des revendications 1 à 8, au moins une substance active dermatologique dans une quantité effective.

Revendications pour l'Etat contractant suivant: GR

1. Emulsion silicone/eau, caractérisée par le fait qu'elle comporte en milieu aqueux :

1°/un organopolysiloxane à fonction diester présentant, par molécule, au moins un motif de formule (I) suivante :

$$ZR_aSiO_{\frac{3-a}{2}} \qquad (I)$$

dans laquelle :

Z est un radical de formule :

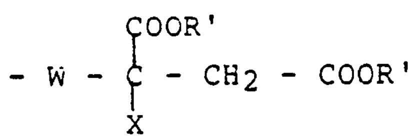

dans laquelle :

. les symboles R′, identiques ou différents, sont choisis parmi les radicaux hydrocarbonés saturés monovalents en $C_1$-$C_{12}$, les radicaux alcoxyalkyle en $C_2$-$C_{12}$ et les radicaux aryle, alkylaryle et arylalkyle en $C_6$-$C_{12}$;

. le symbole X est choisi parmi un atome d'hydrogène et le radical méthyle;

. le symbole W est choisi parmi une liaison covalente et un radical alkylène, linéaire ou ramifié, ayant de 1 à 4 atomes de carbone;

. les symboles R, identiques ou différents, sont choisis parmi les radicaux alkyle en $C_1$-$C_{20}$, vinyle, phényle et trifluoro-3,3,3 propyle, hydroxyle, à la condition qu'un seul des radicaux R par atome de silicium soit un hydroxyle;

a est choisi parmi 0, 1 et 2;

2°/une quantité efficace pour former une émulsion d'au moins un émulsionnant choisi parmi les agents tensio-actifs anioniques, cationiques, amphotères et non-ioniques.

2. Emulsion selon la revendication 1, caractérisée par le fait que les agents tensio-actifs anioniques sont choisis parmi les alkylbenzène sulfonates, les alkylsulfates, les alkyléthersulfates, les alkylaryléthersulfates et les dioctylsulfosuccinates de métaux alcalins.

3. Emulsion selon la revendication 1, caractérisée par le fait que les agents tensio-agents cationiques sont choisis parmi les halogénures d'ammonium quaternaires et les sels d'ammonium quaternaires polyéthoxylés.

4. Emulsion selon la revendication 1, caractérisée par le fait que les agents tensio-actifs amphotères sont choisis parmi les N-alkyl($C_{10}$-$C_{22}$) bétaïnes, les N-alkyl($C_{10}$-$C_{22}$)sulfobétaïnes, les N-alkyl($C_{10}$-$C_{22}$)-amidobétaïnes, les alkyl($C_{10}$-$C_{22}$) imidazolines et les dérivés de l'asparagine.

5. Emulsion selon la revendication 1, caractérisée par le fait que les agents tensio-actifs non-ioniques sont choisis parmi les acides gras polyéthoxylés, les esters de sorbitan, les esters de sorbitan polyéthoxylés, les alkylphénols polyéthoxylés, les alcools gras polyéthoxylés, les amides gras polyéthoxylés ou polyglycérolés, les alcools et α-diols polyglycérolés.

6. Emulsion selon l'une quelconque des revendications 1 à 5, caractérisée par le fait que dans la formule (I), les symboles W et X sont choisis parmi :

. W est $CH_2$ et X est H;

. W est $-(CH_2)_3-$ et X est H;

. W est 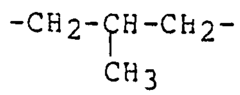 et X est H;

. W est une liaison de valence et X est méthyle.

7. Emulsion selon l'une quelconque des revendications 1 à 6, caractérisée par le fait que l'organopolysiloxane à fonction diester est choisi parmi ceux de formule :

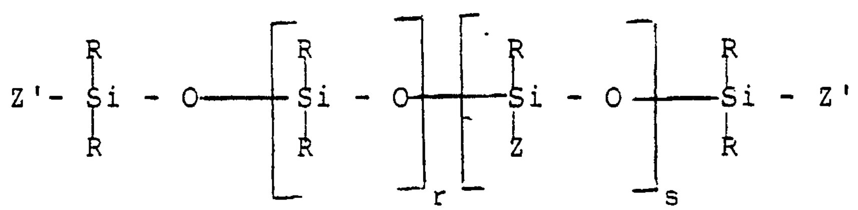 (II)

dans laquelle :

- les symboles R et Z ont la signification donnée dans la revendication 1;
- les symboles Z′, identiques ou différents, sont choisis parmi les radicaux R et Z;
- r est un nombre entier compris entre 0 et 500 inclusivement;
- s est un nombre entier choisi entre 0 et 50 inclusivement, et, si s est 0, au moins un des deux symboles Z′ est Z;

et ceux de formule :

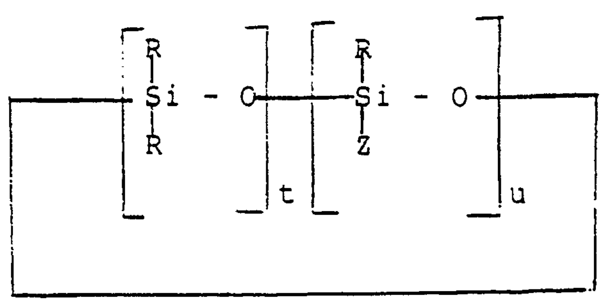 (III)

dans laquelle :

R et Z ont la même signification que dans la revendication 1;
u est un nombre entier compris entre 1 et 20 inclus; et
t est un nombre entier compris entre 0 et 20 inclus;
t + u est supérieur ou égal à 3.

8. Emulsion selon la revendication 7, caractérisée par le fait que dans la formule de l'organosiloxane à fonction diester :

- R et R′ sont méthyle;
- r est compris entre 5 et 50 inclus;
- s est compris entre 1 et 20 inclus;
- t + u est compris entre 3 et 10 inclus.

9. Emulsion selon l'une quelconque des revendications précédentes, caractérisée par le fait qu'elle comporte :

- 100 parties en poids de l'organopolysiloxane à fonction diester;
- 1 à 300 parties en poids d'au moins un agent tensio-actif;
- 5 à 2 000 parties d'eau.

10. Utilisation de l'émulsion telle que définie dans l'une quelconque des revendications 1 à 9, pour le traitement des fibres textiles naturelles ou synthétiques.

11. Utilisation de l'émulsion telle que définie dans l'une quelconque des revendications 1 à 9, pour le traitement des fibres kératiniques.

12. Utilisation de l'émulsion telle que définie dans l'une quelconque des revendications 1 à 9, pour l'enduction de supports souples, en particulier l'enduction de supports cellulosiques.

13. Utilisation de l'émulsion telle que définie dans l'une quelconque des revendications 1 à 9, pour le traitement cosmétique ou dermatologique des cheveux ou de la peau.

14. Composition cosmétique destinée au traitement des cheveux ou de la peau et se présentant sous forme d'une émulsion aqueuse silicone/eau, caractérisée par le fait que l'émulsion est telle que définie dans l'une quelconque des revendications 1 à 9.

15. Composition selon la revendication 14, caractérisée par le fait qu'elle comprend :

(i) au moins un organopolysiloxane à fonction diester présentant par molécule au moins un motif de formule (I) suivant :

$$ZR_aSiO_{\frac{3-a}{2}} \quad (I)$$

dans laquelle :

Z est un radical de formule :

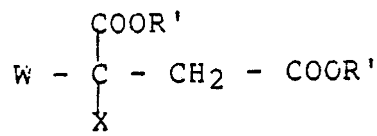

dans laquelle :

. les symboles R', identiques ou différents, sont choisis parmi les radicaux hydrocarbonés saturés monovalents en $C_1$-$C_{12}$, les radicaux alcoxyalkyle en $C_2$-$C_{12}$, et les radicaux aryle, alkylaryle et arylalkyle en $C_6$-$C_{12}$;

. le symbole X est choisi parmi un atome d'hydrogène et le radical méthyle;

. le symbole W est choisi parmi une liaison covalente et un radical alkylène, linéaire ou ramifié, ayant de 1 à 4 atomes de carbone;

. les symboles R, identiques ou différents, sont choisis parmi les radicaux alkyle en $C_1$-$C_{20}$, vinyle, phényle et trifluoro-3,3,3 propyle, hydroxyle, à la condition qu'un seul des radicaux R par atome de silicium soit un hydroxyle;

(ii) et au moins un halogénure d'ammonium quaternaire comportant au moins une chaîne grasse en $C_{10}$-$C_{30}$.

16. Composition selon la revendication 15, caractérisée par le fait que l'halogénure d'ammonium quaternaire est choisi parmi le chlorure de distéaryldiméthylammonium et le chlorure de béhényl triméthylammonium.

17. Composition selon la revendication 15, caractérisée par le fait qu'elle renferme dans un milieu aqueux 0,01 à 15% en poids d'halogénure d'ammonium quaternaire dont un au moins des groupements alkyle est un groupement en $C_{10}$-$C_{30}$ et au moins un polyorganosiloxane tel que défini dans l'une quelconque des revendications 1, 6, 7 et 8, dans des proportions comprises entre 0,01 et 15% et de préférence entre 0,02 et 10% en poids.

18. Composition selon l'une quelconque des revendications 14 à 17, caractérisée par le fait qu'elle contient également un solvant cosmétiquement acceptable choisi parmi les alcools en $C_1$-$C_4$.

19. Composition selon l'une quelconque des revendications 14 à 18, caractérisée par le fait qu'elle contient en plus au moins un adjuvant cosmétiquement acceptable choisi parmi les parfums, les colorants, les agents conservateurs, les produits hydratants, les agents séquestrants, les agents filtrants solaires, les agents moussants, les agents de conditionnement, les agents épaissisants, les stabilisateurs de mousse, les propulseurs.

20. Composition selon l'une quelconque des revendications 14 à 19, caractérisée par le fait qu'elle contient en plus, au moins un polymère cationique et au moins un polymère anionique ayant un poid moléculaire compris entre 500 et 3.000.000.

21. Composition selon l'une quelconque des revendications 14 à 20, caractérisée par le fait que son pH est ajusté entre 3 et 10.

22. Composition selon l'une quelconque des revendications 14 à 21, caractérisée par le fait qu'elle contient des épaississants choisis parmi la gomme de xanthane, la gomme de guar ou ses dérivés, la

gomme arabique ou la gomme de caroube, l'alginate de sodium, les dérivés de cellulose, les dérivés d'acide polyacrylique, des mélanges d'acide phosphorique et d'amides; ou bien le produit résultant de l'interaction ionique d'un polymère cationique constitué par un copolymère de cellulose ou d'un dérivé de cellulose greffé par un sel de monomère hydrosoluble d'ammonium quaternaire et d'un polymère anionique carboxylique ayant une viscosité capillaire absolue dans le diméthylformamide ou le méthanol, à une concentration de 5% et à 30 °C, égale ou inférieure à $30 \times 10^{-3}$ Pa.s.

23. Application de la composition telle que définie dans l'une quelconque des revendications 14 à 22, comme shampooing, comme produit après-shampooing, produit à rincer appliqué avant ou après shampooing, avant ou après coloration ou décoloration, avant ou après permanente ou défrisage, comme lotion de mise en plis ou de brushing, comme composition restructurante, comme additif aux permanentes.

24. Procédé de traitement cosmétique des cheveux ou de la peau, caractérisé par le fait que l'on applique sur les cheveux ou sur la peau au moins une composition telle que définie dans l'une quelconque des revendications 14 à 22.

25. Composition dermatologique, caractérisée par le fait qu'elle se présente sous la forme d'une émulsion aqueuse silicone/eau telle que définie dans l'une quelconque des revendications 1 à 9 et qu'elle contient, en outre, une substance active dermatologique.

Office européen des brevets

# RAPPORT DE RECHERCHE EUROPEENNE

Numero de la demande

EP 89 40 2629

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | US-A-4 405 469 (HAFNER et al.)<br>* Revendications *<br>----- | 1-25 | A 61 K 7/06<br>C 08 G 77/38<br>D 06 M 15/643 |
| | | | **DOMAINES TECHNIQUES RECHERCHES (Int. Cl.5)** |
| | | | A 61 K<br>C 08 G |

Le présent rapport a été établi pour toutes les revendications

| Lieu de la recherche | Date d'achèvement de la recherche | Examinateur |
|---|---|---|
| LA HAYE | 14-12-1989 | WILLEKENS G.E.J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)